Europäisches Patentamt

European Patent Office

Office européen des brevets

(11) Publication number: **0 315 840 B1**

# EUROPEAN PATENT SPECIFICATION

(45) Date of publication of patent specification: **21.10.92**

(51) Int. Cl.5: **A61L  15/24**, A61L 25/00,
C08F 289/00, C08F 283/06,
C08L 51/08

(21) Application number: **88117993.1**

(22) Date of filing: **28.10.88**

(54) Method of manufacturing hydrogle dressing films.

(30) Priority: **11.11.87 PL 268780**

(43) Date of publication of application:
**17.05.89 Bulletin  89/20**

(45) Publication of the grant of the patent:
**21.10.92 Bulletin  92/43**

(84) Designated Contracting States:
**AT CH DE FR IT LI NL**

(56) References cited:
**WO-A-84/04098**
**US-A- 4 556 056**

**ABSTRACT BULLETIN OF THE INSTITUTE OF
PAPER CHEMISTRY, vol. 34, no. 2, October
1963, page 197, abstract 713; B.H. THEWLIS:
"New products from wheat flour and wheat
starch", & J. APPL. CHEM. 13, NO. 6: 249-53
(JUNE 1963)**

(73) Proprietor: **Politechnika Slaska im. Wincen-
tego Pstrowskiego
ul. Krzywoustego 7
P-44-101 Gliwice(PL)**

(72) Inventor: **Jedlinski, Zbigniew
ul. Karolinki 16/1
P-44-110 Gliwice(PL)**
Inventor: **Lukaszczyk, Jan
ul. Zorska 20
P-43-180 Orzesze(PL)**
Inventor: **Pradellok, Witold
ul. Bekasa 1/37
P-44-100 Gliwice(PL)**

(74) Representative: **Hansen, Bernd, Dr.rer.nat. et
al
Hoffmann, Eitle & Partner Patentanwälte Ar-
abellastrasse 4 Postfach 81 04 20
W-8000 München 81(DE)**

## Description

The subject of the invention is a method of manufacturing hydrogel dressing films.

It is known in surgical technique to use material of hydrogel film type for dressing oozing or graining wounds. Known dressing materials of this type are manufactures by means of polymerization, in an aqueous medium, of hydrophilic monomers, in the presence of natural polymers, capable of forming gels, such as, e.g. polisaccharides or simple proteins. Dressings obtained in this way may be manufactured in form of water-swollen hydrogel films, as described in DE-A-2725261 or in form of film or powder - dried, but capable of re-swelling in aqueous medium, as presented in the Polish patent specification No 118053 and the EP-A-48325. Known methods of manufacturing these materials require applying burdensome processes of removing residue initiator, which usually is a system of persulphate tertiary amine and unreacted monomer, left over from the polymerization process. Also known are methods of radiation induced polymerization allowing to obtain hydrogel films free of chemical initiator additions, as presented in the Polish patent specification No 128392.

Similarly with the methods mentioned before, this process has a deficiency in form of the presence of monomer residues in the final product, and the products of radiolysis, and these contaminants must be removed before the hydrogel film may be used as dressing material.

It has been found that the mentioned burdensome technological processes may be omitted using the method of the invention, consisting in manufacturing hydrogel dressing films by means of polymerization of hydrophilic monomer present in an aqueous solution with natural polymers or other hydrophilic monomers, using an hydrogen peroxide as initiator and 1-hydroxy-2,3-epoxypropane as a modifier.

Hydrogel films obtained in this way do not contain any chemical initiator residues of persulphate/amine type nor product of its decomposition. Certain amounts of hydrogen peroxide, used as initiator in the method of the invention are harmless and do not need removing as they dissociate into water and oxygen.

1-hydroxy-2,3-epoxypropane used as a modifier in the method of the invention reacts with the components of the polymerization mixture and the resulting polymers are very flexible with good hydrophilic conditions and preferable water retention. The products of 1-hydroxy-2,3-epoxypropane conversion, i.e. glycerine and polyglycerines are nontoxic and as such do not require removal.

Addition of this modifying agent gives better effects than hitherto used modifiers in form of polyglycols in known methods of the above mentioned solutions.

Example I. 4.8 g of agar was dissolved in hot 200 ml of distilled water, blown with inert gas, free of oxygen. Solution of 24 g acrylcamide, 0.48 g methylenobis-acrylamide and 2.4 g 1-hydroxy-2,3-epoxypropane in 80 ml of distilled water has been added to the previously obtained solution. The mixture was agitated and heated to the temperature of 45-50°C, then 0.64 g of 30% aqueous solution of hydrogen peroxide/Perhydrol/ was added. Dismountable polymerization moulds were filled with the effecting solution and then the moulds were placed for 2 hours in an oven at a temperature of 75°C. On cooling, thus manufactured hydrogel films, their thickness and dimensions corresponding with the dimensions of moulds, are rinsed with distilled water and left in that water for 15 minutes. After that time the monomer residue in the film does not exceed $10^{-6}$ g/l. Hydrogel film, packed in sealed polyethylene bags, sterilized with the use of temperature of ethylene oxide, is a flexible transparent, highly adherent wound dressing, easily disposable after a time, as it does not stick to the wound or injured skin.

Example II. A solution of 24 g acrylamide, 0.48 methylenobisacrylamide, 3.2 g agar, 4.8 g 1-hydroxy-2-3-epoxypropane, 1.6 g high-molecular polyethylene oxide in 286 ml water was prepared and polymerized. Further proceedings to obtain dressing materials as described in Example I.

Example III. Solution of 16 g acrylamide, 0.32 g methylenobisacrylamide, 4.8 g agar, 4.8 g 1-hydroxy-2-3-epoxypropane and 0.75 g Perhydrol in 298 ml of physiological salt solution was prepared and polymerized as described in Example I, then films were rinsed with physiological salt solution.

Example IV. Solution of 32 g acrylamide, 0.32 g methylenobisacrylamide, 3.2 g 1-hydroxy-2-3-epoxypropane, 7.2 g agar, 0.64 g Perhydrol in 277 ml distilled water, was prepared and polymerized in the way described in Example I.

The foil obtained was about 3 mm thick and after rinsing it was seasoned in the air during 8-12 hours, till its thickness was reduced to 1.5-2.0 mm. Freed of a part of water, the foils are sufficiently flexible to be used on wounds, after being sterilized. They also may be saturated with disinfecting or therapeutical agents soluble in water.

## Claims

1.  Method of manufacturing hydrogel dressing foils by means of polymerization of hydrophilic monomers in aqueous solution in the presence of natural polymers capable of gelling and optional other additives, characterised in that polymerization is performed in the presence of hydrogen peroxide as initiator and 1-hydroxy-2,3-epoxypropane as modifier.

2. Method as claimed in claim 1, characterised in that the concentration of hydrogen peroxide in the solution amounts to 0.02-0.15% by weight, preferably 0.05-0.10% by weight.

3. Method as claimed in claim 1, characterised in that the concentration of 1-hydroxy-2,3-epoxypropane in the polymerization solution amounts to 0.5-5.0%, preferably 1-2% by weight.

4. Method as claimed in claim 1, characterised in that polymerisation is performed at a temperature of 65 - 75°C.

**Patentansprüche**

1. Verfahren zum Herstellen von Hydrogel-Verbandfolien mittels Polymerisation von hydrophilen Monomeren in wässriger Lösung in Anwesenheit von natürlichen Polymeren, die sich dazu eignen, zu gelieren, und gewünschtenfalls anderen Additiven, dadurch **gekennzeichnet,** dass die Polymerisation in Anwesenheit von Wasserstoffperoxid als Initiator und 1-Hydroxy-2,3-epoxypropan als Modifikationsmittel durchgeführt wird.

2. Verfahren nach Anspruch 1, dadurch **gekennzeichnet,** dass die Wasserstoffperoxid-Konzentration in der Lösung 0,02 bis 0,15 Gew.%, vorzugsweise 0,05 bis 0,10 Gew.%, beträgt.

3. Verfahren nach Anspruch 1, dadurch **gekennzeichnet,** dass die 1-Hydroxy-2,3-epoxypropan-Konzentration in der Polymerisationslösung 0,5 bis 5 Gew.%, vorzugsweise 1 bis 2 Gew.%, beträgt.

4. Verfahren nach Anspruch 1, dadurch **gekennzeichnet,** dass die Polymerisation bei einer Temperatur von 65 bis 75°C durchgeführt wird.

**Revendications**

1. Méthode de préparation de feuilles d'hydrogel pour pansement, par polymérisation de monomères hydrophiles en solution aqueuse, en présence de polymères naturels, capables de gélifier, et d'autres additifs facultatifs, caractérisée en ce que la polymérisation est effectuée en présence de peroxyde d'hydrogène, en tant qu'initiateur, et de hydroxy-1-époxy-2,3-propane en tant que modificateur.

2. Méthode selon la revendication 1, caractérisée en ce que la concentration en peroxyde d'hydrogène, dans la solution, s'élève à 0.02-0,15% en poids, de préférence 0,05-0,10% en poids.

3. Méthode selon la revendication 1, caractérisée en ce que la concentration en hydroxy-1-époxy-2,3-propane, dans la solution de polymérisation, s'élève à 0,5-5,0%, de préférence 1-2% en poids.

4. Méthode selon la revendication 1, caractérisée en ce que la polymérisation est effectuée à une température de 65-75°C.